# EUROPEAN PATENT APPLICATION

(11) **EP 1 609 442 A1**
(43) Date of publication of application: **28.12.2005**
(21) Application number: 04724371.2
(22) Date of filing: 30.03.2004
(51) Int. Cl.: A61F 2/28

(54) **MATERIAL FOR REPAIRING BIOLOGICAL TISSUES AND PROCESS FOR PRODUCING THE SAME**

(30) Priority: 31.03.2003 JP 2003095017
(71) Applicant: Olympus Corporation, Tokyo 151-0072 (JP)
(72) Inventor: HAKAMATSUKA, Yasuharu, Akishima-shi, Tokyo 196-0033 (JP); TAKAMIYA, Yuji;, Kobe-shi, Hyogo 657-0068 (JP); SADAMORI, Katsuya, Hino-shi, Tokyo 191-0022 (JP)
(74) Representative: von Hellfeld, Axel
(86) International application number: PCT/JP2004/004550
(87) International publication number: WO 2004/087020

(57) **Abstract**

An object is to promote the growth of adhered cells and improve adhesiveness of stem cells. A cylindrical scaffold made of a porous calcium phosphate (for example, beta-TCP) material has a form in which a plurality of through-holes extending in a single direction are separated from each other by partition wall members having an almost uniform thickness. The material for repairing biological tissues wherein each through-hole has a quadrangular shape, and each partition wall member has a plurality of concavities and a plurality of pores in the surface, and a process for producing the same are provided.

## Description

### TECHNICAL FIELD

The present invention relates to a material for repairing biological tissues used for repairing a defect in biological tissues, and a process for producing the same.

### BACKGROUND ART

Recently, it has become possible to repair a defect in biological tissue such as bone caused by osteoncus extraction, trauma, or the like by regenerating the bone by filling a material for repairing biological tissues such as a bone substitute or the like. For such a bone substitute, hydroxyapatite (HAP) and tricalcium phosphate (TCP) are known. However, from the viewpoint of leaving no foreign matter inside the body, a scaffold made of a porous calcium phosphate material, for example, such as β-TCP is used. If the β-TCP is left in contact with bone cells of a defect part of bone, so-called remodeling is performed in which osteoclasts eat the β-TCP, and osteoblasts form a new bone. That is, the bone-repairing material filled in the defect part of the bone is replaced by autologous bone as time goes by.

Meanwhile, in order to increase the speed in repairing the defect part of the bone after the operation, it has been proposed to use a product for repairing biological tissues such as a cultured bone, produced by soaking the bone substitute in bone marrow liquid collected from the patient, and then culturing bone marrow mesenchymal stem cells contained in the bone marrow liquid with the bone substitute. Since the defect part of bone is filled with the bone-repairing product including a lot of bone marrow mesenchymal stem cells that have proliferated by being cultured using the bone substitute as a scaffold, the number of days required until the bone-repairing product is replaced by the autologous bone can be greatly shortened compared to a method in which cells are made to proliferate inside the body after an operation (refer to: Uemura and two others, "Tissue engineering in bone using biodegradable β-TCP porous material - a new material strengthened in vivo Osferion", Medical Asahi, The Asahi Shimbun Company, October 1, 2001, Vol. 30, No. 10, p. 46-49).

### DISCLOSURE OF THE INVENTION

However, even if a porous material is used for the bone substitute, the bone marrow mesenchymal stem cells stay only on the surface of the bone substitute, and it has been difficult to sufficiently adhere the bone marrow mesenchymal stem cells to the inside of the bone substitute. Moreover, even if the cells are adhered onto the bone substitute, there has been a problem in that components required for the cell growth do not permeate into the inside. Meanwhile, there whas been a probrem in that cell waste matter collects in the vicinity of the cells so that smooth exchange of the cell waste matter is hampered.

In view of the above probrems, an object of the present invention is to providea material for repairing biological tissues with which the growth of adhered cells is promoted, and to which the adhesiveness of stem cells is high, and to provide a process for producing the same.

In order to solve the above problems, the present invention employs the following solution.

The material for repairing biological tissues of the present invention has a form in which a plurality of through-holes extending in a single direction are separated from each other by partition wall members having an almost uniform thickness.

Since the material for repairing biological tissues has the abovementioned form, a large surface area can be ensured, and more cells can be adhered on the surface. Moreover, cells can be readily adhered to the inside of the repairing material through the through-holes, and cell waste matters can be readily exchanged through the through-holes.

The material for repairing biological tissues may be in a honeycomb shape.

The present invention is the material for repairing biological tissues, wherein preferably at least one of concavities and pores are formed in the partition wall member.

A process for producing a material for repairing biological tissues of the present invention comprises: a step for mixing a raw material formed in a slurry form, with granular solid bodies having a melting point lower than a sintering temperature; a step for supplying the raw material mixed with the solid bodies into a mold, and forming a molded article in which a plurality of through-holes are separated from each other by partition wall members having an almost uniform thickness; and a step for sintering the molded article.

The molded article may be a honeycomb molded article that is formed into a honeycomb shape.

### BRIEF DESCRIPTION OF THE DRAWINGS

FIG. 1 shows the appearance of a material for repairing biological tissues in an embodiment of the present invention.
FIG. 2A shows an example of a cross-section taken along the line II-II of FIG. 1.
FIG. 2B shows another example of a cross-section taken along the line II-II of FIG. 1.
FIG. 3 shows a production flow of the material for repairing biological tissues, in the embodiment of the present invention.
FIG. 4 shows the appearance of a honeycomb mold used for honeycomb molding in the embodiment of the present invention.
FIG. 5 shows the cross-section of a honeycomb molded article containing solid bodies in the embodiment of the present invention.

### BEST MODE FOR CARRYING OUT THE INVENTION

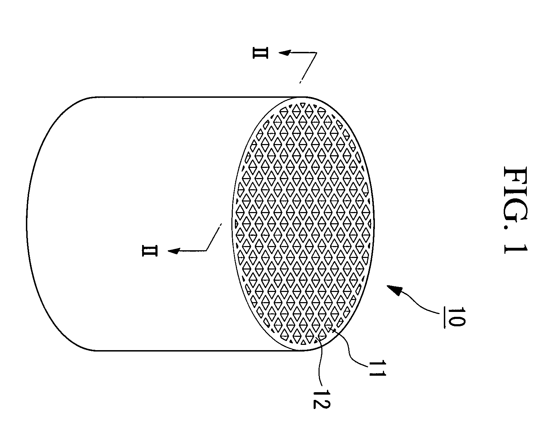

Hereunder is a description of a material for repairing biological tissues according to an embodiment of the present invention, with reference to FIG. 1 to FIG. 5.

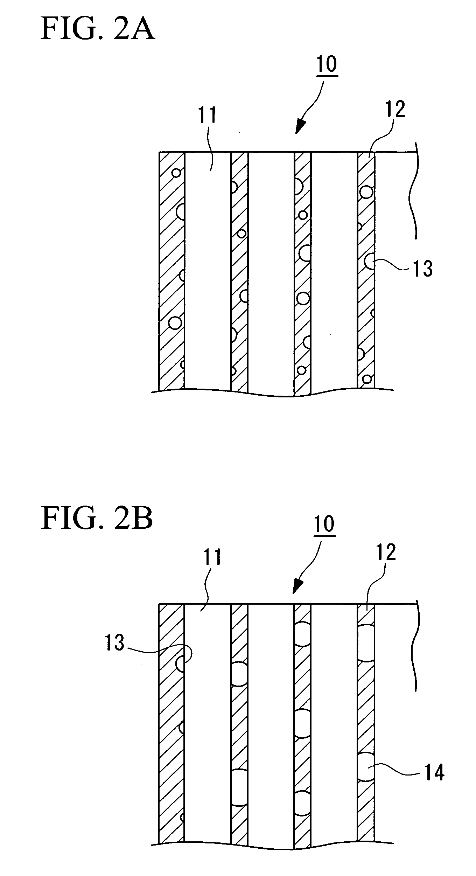

As shown in FIG. 1, a bone substitute (material for repairing biological tissues) 10 according to the present embodiment is a cylindrical scaffold made of a porous calcium phosphate (for example, β-TCP) material in which a plurality of through-holes 11 extending in a single direction are separated from each other by partition wall members 12 having an almost uniform thickness (for example, about 0.05 mm to 0.5 mm) to give a honeycomb shape. Each through-hole 11 is mainly formed into a quadrangular shape, and as shown in FIG. 2, each partition wall member 12 has a plurality of concavities 13 and a plurality of pores 14 in the surface.

Next is a description of a process for producing the bone substitute 10 according to the present embodiment comprising the above structure.

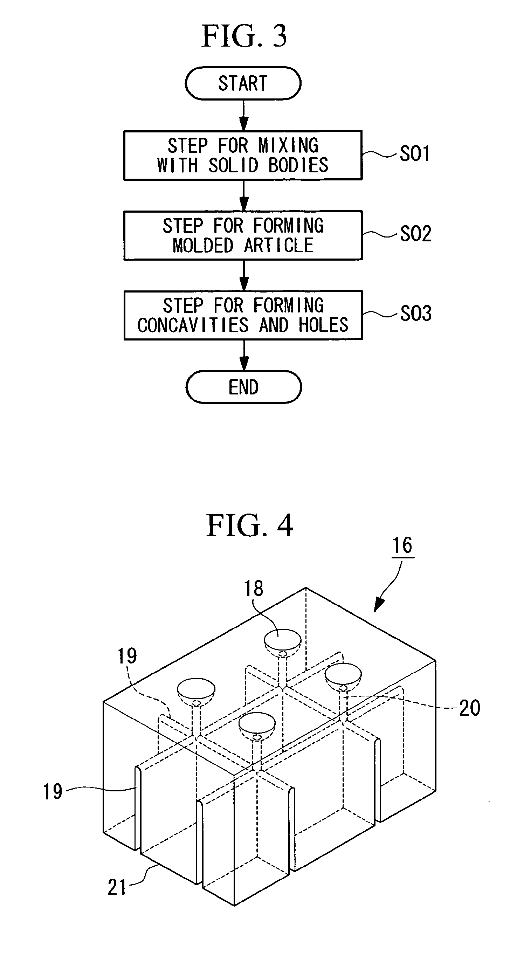

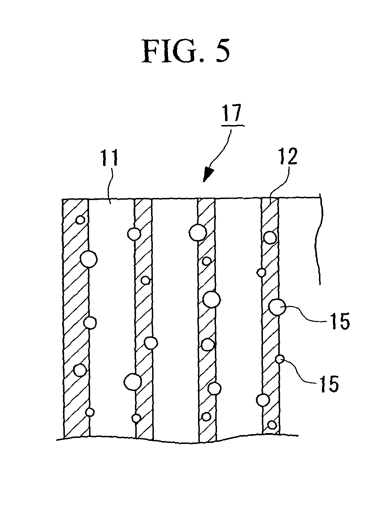

As shown in FIG. 3, the production process for the bone substitute 10 comprises: a step (S01) for mixing a raw material formed in a slurry form, with granular wax beads (solid body) 15 as shown in FIG. 5 having a melting point lower than a sintering temperature; a step (S02) for casting the raw material mixed with the wax beads 15 in a honeycomb mold (mold) 16 as shown in FIG. 4, so as to form a honeycomb molded article 17 as shown in FIG. 5 in which a plurality of through-holes 11 are separated from each other by partition wall members 12 having an almost uniform thickness; and a step (S03) for sintering the honeycomb molded article 17. Hereunder is a description of the respective steps.

In step (S01) for mixing with the wax beads 15, firstly, for example by a method disclosed in Japanese Unexamined Patent Application, First Publication No. Hei 5-237178, an aqueous foamed slurry which has been mixed and foamed, is adjusted and mixed with granular molded articles made from β-TCP so as to make a raw material which is formed in a slurry form.

The wax beads 15 are then mixed into the raw material.

Next is a description of a step (S02) for forming the honeycomb molded article 17.

In this step, the raw material mixed with the wax beads 15 is supplied to the honeycomb mold 16 shown in FIG. 4 to make the honeycomb molded article 17 shown in FIG. 5.

In the top of the honeycomb mold 16 of FIG.4, is formed raw material inlets 18 that have been formed in a funnel shape. Meanwhile, the inside at the bottom is formed with slits 19 extending respectively in parallel with the respective sides of the honeycomb mold 16, and having for example a width of 0.1 mm. The bottom ends of the raw material inlets 18 and one end of the slits 19 are communicated through passages 20.

The raw material injected from the raw material inlet 18 permeates into each slit 19 through the passages 20. Then, the honeycomb molded article 17 in honeycomb shape formed with the through-holes 11 in a quadrangular shape and the partition wall members 12 separating the respective through-holes 11, is discharged from the bottom surface 21 of the honeycomb mold 16. In this case, as shown in FIG. 5, the cross-section of the honeycomb molded article 17 has a structure that includes the wax beads 15 in the partition wall members 12.

Next is a description of a step (S03) for sintering the honeycomb molded article 17.

The honeycomb molded article 17 is sintered after drying, for example in a method disclosed in Japanese Unexamined Patent Application, First Publication Hei 5-237178. At this time, since the melting point of the wax beads 15 contained in the honeycomb molded article 17 is lower than the sintering temperature, then after being melted by heating during the sintering, the wax beads 15 run out to the outside of the partition wall members 12 and are removed from the through-holes 11 to outside of the honeycomb molded article 17.

In this way, as shown in FIG. 2A and FIG. 2B, concavities 13 and pores 14 are formed in the surface and the interior of the partition wall members 12.

Here is a description of a case where cells are added to the bone substitute 10 obtained in this way, so as to make the bone-repairing product.

Firstly, bone marrow liquid is extracted from the ilium or the like, and the bone marrow liquid is concentrated by an appropriate process such as centrifugation. Then, a medium to which has been added growth factors such as MEM (Minimal Essential Medium) and FBS (Fetal Bovine Serum) is supplied to a culturing container and mixed with the bone marrow liquid, and primary culturing is performed so as to make a cell concentrate containing mesenchymal stem cells with unwanted components removed.

Next, the cell concentrate is permeated throughout the bone substitute 10 that has been made by the abovementioned method. Then, it is introduced into the bone forming medium containing dexamethasone or the like serving as a factor for inducing differentation into osteoblasts, so as to perform secondary culturing.

In this manner, a bone-repairing product furnished with a bone forming function, and having the produced bone matrix, and osteoblasts differentiated from mesenchymal stem cells, is obtained.

According to the bone substitute 10, since it is formed by a honeycomb mold, a large surface area can be ensured and the diameter of the holes can be readily controlled, enabling optimum size through-holes to be freely made. Moreover, since the through-holes 11 are provided, the mesenchymal stem cells can be readily adhered to the inside of the bone substitute 10, and waste matter can be readily exchanged with respect to the mesenchymal stem cells through the through-holes. Since not only holes but also concavities 13 are formed for the porous material, the mesenchymal stem cells are readily adhered onto the surface of the partition wall members 12, and a sufficient amount of cells can be adhered. Furthermore, not only the distribution along the direction of the through-holes 11 is improved, but also the communication between the plurality of the through-holes 11 is improced by the pores 14 so as to enhance the accumulation of the mesenchymal stem cells on the partition wall members 12, and the exchange of waste matter.

The technical scope of the present invention is not limited by the abovementioned embodiment, and various modifications can be made thereto without departing from the gist of the present invention.

For example, the shape of the through-holes is not limited to the quadrangular shape, and may be a hexagonal shape as with honeycomb shape, a triangular shape, or a circular shape. Moreover, the size of the respective through-holes is not necessarily uniform, and may be nonuniform.

Furthermore, the solid body is not limited to wax beads, and may be plastic beads or any other form as long as it melts at a temperature below the sintering temperature. Moreover the shape of the solid body may be spherical or angular.

Furthermore, the body fluid is not limited to bone marrow liquid, and may be peripheral blood or cord blood, as long as it contains somatic cells such as ES cells, somatic stem cells, bone cells, cartilagenous cells, or nerve cells. The biological tissue also is not necessarily bone tissue, and it is possible to use the present invention for regenerating any arbitrary biological tissue such as cartilaginous tissue, muscular tissue, or subcutaneous tissue.

For the material for repairing biological tissues, any material may be used as long as it has an affinity with the biological tissue, more preferably it has bioabsorbency. The material may also be porous.

The porous material is not necessarily β-TCP, and provided is can form a slurry, it may be any material such as calcium phosphate ceramics, collagen, polylactic acid, or combinations thereof.

### [Example]

Hereunder is a description of an example of using the process for producing the material for repairing biological tissues according to the above embodiment.

Using bone substitute made by the process for producing material for repairing biological tissues according to the above embodiment, a cultured rat bone was made as the bone-repairing product.

Firstly, the bone marrow liquid was extracted from a rat, and primary culturing was performed in a T-flask for 10 days, to generate cultured cells containing bone marrow mesenchymal stem cells with unwanted components removed.

The cultured cells were trypsin treated, and then disseminated onto the bone substitute according to the present embodiment. The medium was then mixed with dexamethasone in addition to MEM and FBS, to initiate the differentiation of the stem cells. Then, secondary culturing was performed for about 2 weeks.

In this manner, a bone-repairing product containing osteoblasts that had been differentiated from the bone marrow mesenchymal stem cells was obtained.

The bone-repairing product was grafted subcutaneously into the rat, and taken out therefrom 4 weeks later. As a result, the generation of new bone tissue was confirmed.

### INDUSTRIAL APPLICABILITY

According to the material for repairing biological tissues of the present invention, when the cells permeated into the through-holes are adhered to the surface of the partition wall members, they are captured by the concavities or pores formed in the partition wall members. As a result, the adhesiveness of the cells is increased so that a sufficient amount of cells can be adhered.

According to the process for producing the material for repairing biological tissues of the present invention, since the surface area of the material for repairing biological tissues is increased, and the cells can be readily adhered, it becomes possible to produce the material for repairing biological tissues wherein a large number of cells can be adhered onto the surface, and cell waste matter can be smoothly exchanged through the through-holes. Furthermore, since the through-holes can be readily controlled at the time of the molding, it becomes possible to provide an organism to be grafted having an optimum form.

## Claims

1. A material for repairing biological tissues has a form in which a plurality of through-holes extending in a single direction are separated from each other by partition wall members having an almost uniform thickness.

2. A material for repairing biological tissues according to claim 1, wherein at least one of concavities and pores are formed in said partition wall member.

3. A material for repairing biological tissues according to claim 1, having a honeycomb shape.

4. A process for producing a material for repairing biological tissues comprising:
a step for mixing a raw material formed in a slurry form, with granular solid bodies having a melting point lower than a sintering temperature;
a step for supplying said raw material mixed with said solid bodies into a mold, and forming a molded article in which a plurality of through-holes are separated from each other by partition wall members having an almost uniform thickness; and
a step for sintering said molded article.

5. A process for producing a material for repairing biological tissues according to claim 4, wherein said molded article is formed into a honeycomb shape.
